# EUROPÄISCHE PATENTANMELDUNG

(11) **EP 1 260 926 A2**
(43) Veröffentlichungstag der Anmeldung: **27.11.2002**
(21) Anmeldenummer: 02010667.0
(22) Anmeldetag: 13.05.2002
(51) Int. Cl.: G06F 19/00

(54) **System zur Erfassung und Information von regional gruppierten, tagesaktuellen medizinischen Daten**

(30) Priorität: 23.05.2001 DE 10125543
(71) Anmelder: SIEMENS AKTIENGESELLSCHAFT, 80333 München (DE)
(72) Erfinder: Christ, Tilo, 91058 Erlangen (DE); Schmidt, Volker, Dr., 91054 Erlangen (DE); Schneider, Siegfried, Dr., 91056 Erlangen (DE); Schüll, Hans-Dieter, 91085 Weisendorf (DE)

(57) **Zusammenfassung**

Die Erfindung betrifft ein System zur Erfassung und Information von regional gruppierten, tagesaktuellen medizinischen Daten mit Vorrichtungen (4 bis 7) zur Erfassung der medizinischen Daten, mit Vorrichtungen (9 bis 12) zur Übertragung der medizinischen Daten an eine Systemzentrale (8), mit einer Auswertevorrichtung (14) für die medizinischen Daten, mit Vorrichtungen (4 bis 7) zur Abfrage der ausgewerteten medizinischen Daten und mit Vorrichtungen (9 bis 12) zur Übertragung der ausgewerteten medizinischen Daten von der Systemzentrale (8), wobei die Systemzentrale (8) einen Speicher (15) für die erfassten medizinischen Daten und Sollwerte für die medizinischen Daten, und die Auswertevorrichtung (14) einen Komparator zum Vergleich der medizinischen Daten mit den gespeicherten Sollwerten aufweist.

## Beschreibung

Die Erfindung betrifft ein System zur Erfassung und Information von regional gruppierten medizinischen Daten mit einer Auswertevorrichtung. Ein derartiges System dient als Informationsdienst über die regional gruppierte, tagesaktuelle Häufigkeit und Verteilung von Infektionskrankheiten.

Es sind medizinische Expertensysteme bekannt, die mit Wahrscheinlichkeiten arbeiten. Bei diesen wahrscheinlichkeitsbasierten Expertensystemen erfolgt eine Parametrisierung z. B. über statistische Daten, die beispielsweise im Rahmen einer Studie gewonnen wurden. Diese Daten spiegeln keine aktuellen Situationen wieder, und sie werden in den Datenmodellen fest implementiert. In der Konsequenz können solche Expertensysteme nur in Bereichen eingesetzt werden, in denen sich a priori Wahrscheinlichkeiten nicht ändern.

Aus der DE 199 30 250 A1 ist eine Vorrichtung zur Überwachu8ng von Daten insbesondere aus einem elektromedizinischen Implantat bekannt, bei dem es um die Übertragung von Daten aus einem medizinelektronischen Gerät eines individuellen Patienten über ein Mobilfunknetz geht.

In dem Patent US 5,841,975 A ist ein System zur Durchführung von Labortests in einem verteilten System beschrieben, bei dem es um die Erstellung einer Diagnose geht.

Die Erfindung geht von der Aufgabe aus, ein Expertensysteme der eingangs genannten Art zu schaffen, dass auf einfache Weise aktuelle, reale statistische Daten für korrekte Schlussfolgerungen sammelt.

Die Aufgabe wird erfindungsgemäß dadurch gelöst, durch ein System mit Vorrichtungen zur Erfassung der medizinischen Daten, mit Vorrichtungen zur Übertragung der medizinischen Daten an eine Systemzentrale, mit einer Auswertevorrichtung für die medizinischen Daten, mit Vorrichtungen zur Abfrage der ausgewerteten medizinischen Daten und mit Vorrichtungen zur Übertragung der ausgewerteten medizinischen Daten von der Systemzentrale gelöst, wobei die Systemzentrale einen Speicher für die erfassten medizinischen Daten und Sollwerte für die medizinischen Daten, und die Auswertevorrichtung einen Komparator zum Vergleich der medizinischen Daten mit den gespeicherten Sollwerten aufweist.

Durch das "Aufstellen" vieler Datenerfassungspunkte können aktuelle Daten für epidemiologische Kartografierungen gewonnen und verarbeitet werden. Durch dieses System erhält man eine einfache und kostengünstige Unterstützung beispielsweise von Bürgern und Ärzten einer Region, wie beispielsweise eines Stadtteils, einer Stadt, eines Kreises oder eines Bezirks, in Bezug auf Informationen über die aktuelle Verbreitung von ansteckenden Krankheiten und kann dementsprechende Maßnahmen zur Reduzierung und Eindämmung einleiten.

Bei den medizinischen Daten handelt es sich um epidemiologische, aktuelle Daten, wie beispielsweise das Vorliegen einer Masernepidemie innerhalb einer Region. Über eine verteilte Eingabe- und Abfragemöglichkeit für medizinische Daten werden aktuelle Krankheitszustände lokal erfasst und daraus aktuelle epidemiologische Karten errechnet. Kommt eine Anfrage, z.B. wie gerade die Masernhäufigkeit in der Region ist, so liefert das System diesen Wert. Ein Expertensystem kann mit diesen statistischen Daten konfiguriert werden, so dass es in Anfrageergebnisse aktuelles Wissen über regionale Häufungen mit einfließen lassen kann.

In vorteilhafter Weise kann die Auswertevorrichtung ein Expertensystem aufweisen.

Erfindungsgemäß können die Vorrichtungen zur Erfassung der medizinischen Daten im Hause beispielsweise eines Patienten, bei Institutionen und/oder bei ärztlichen Dienststellen angeordnet sein.

Eine einfache Erfassung ergibt sich, wenn die Vorrichtungen zur Erfassung der medizinischen Daten und/oder der ausgewerteten Daten über das Internet (WWW) mit der Systemzentrale kommunizieren. Dabei hat es sich als vorteilhaft erwiesen, wenn die medizinischen Expertensystemen über die Daten aus dem Internet automatisch konfiguriert werden.

Nahezu jeder kann die Daten erfassen und abfragen, wenn die Vorrichtungen zur Erfassung der medizinischen Daten und/oder der ausgewerteten Daten über ein Telefon mit der Systemzentrale, beispielsweise mit einem Call-Center, kommunizieren.

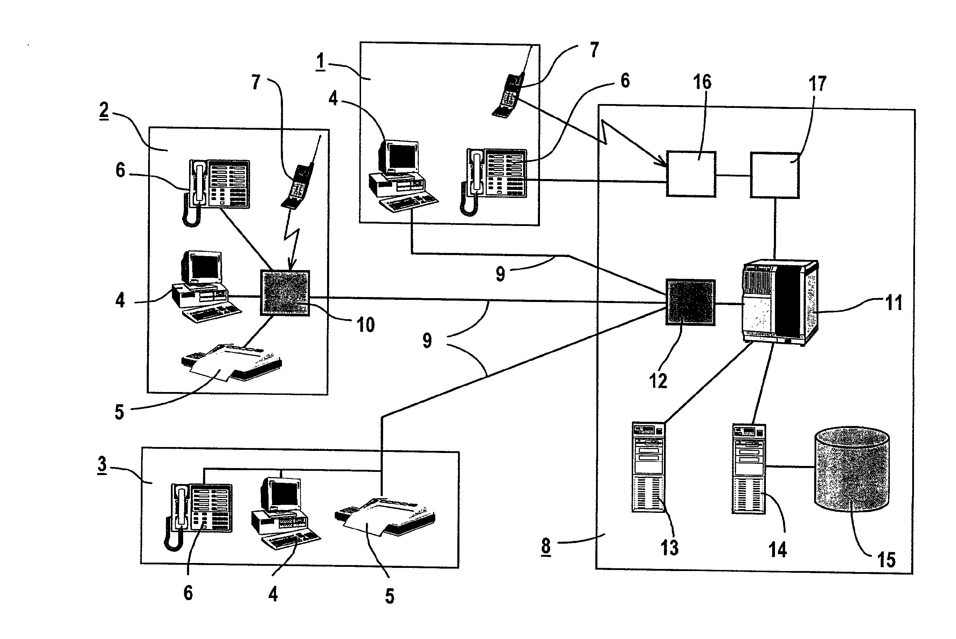

Die Erfindung ist nachfolgend anhand von in der Zeichnung dargestellten Ausführungsbeispielen näher erläutert.

In der Figur ist ein erfindungsgemäßes System zur Erfassung und Information von regional gruppierten, tagesaktuellen medizinischen Daten dargestellt. Zur Erfassung der medizinischen Daten können der Patient in seinem Haus 1, Institutionen 2, wie beispielsweise Schule, Kindergarten, Arbeitgeber o.ä. sowie ärztliche Dienststellen 3, wie beispielsweise Arzt, Apotheke oder Krankenhaus, dienen.

Diese tagesaktuellen Daten können als aktuelle a priori Wahrscheinlichkeiten direkt per Personal Computer 4, per Faxgerät 5 oder per Telefon 6 oder Handy 7 an eine Systemzentrale 8 weitergeleitet werden. Dies kann beispielsweise über ein ISDN-Netz 9 erfolgen, an das die entsprechenden Endgeräte direkt oder über ein ISDN-Interface 10 angeschlossen sind.

In der Systemzentrale 8 ist ein Gateway 11 vorgesehen, das über ein ISDN-Interface 12 an dem ISDN-Netz 9 angeschlossen. An dem Gateway 11 können ein Internet-Proxy-Server 13 zum Zugriff auf das Internet und eine Auswertevorrichtung 14 mit einem Datenspeicher 15 für die tagesaktuellen Daten angeschlossen sein.

Die an die Systemzentrale 8 beispielsweise per Telefon 6 oder Handy 7 weitergeleiteten Daten können von einem Call-Center 16 aufgenommen werden und durch eine Ein- und Ausgabevorrichtung in das System eingegeben werden und auf die gleiche Art auch wieder abgerufen werden.

Die Auswertevorrichtung 14 weist beispielsweise einen Komparator zum Vergleich der Daten mit in dem Datenspeicher 15 gespeicherten Sollwerten und eine Alarmvorrichtung zur Benachrichtigung ausgewählter Empfänger bei Überschreitung der Messwerte von gespeicherten Sollwertgrenzen auf.

An der Systemzentrale 8 sind Empfangsgeräte wie beispielsweise Faxgeräte 5, Personal Computer 4, Telefone 6 oder Handy 7 zur tagesaktuellen Abfrage der ausgewerteten medizinischen Daten angeschlossen.

In der Systemzentrale 6 werden die medizinischen Daten über genormte Telekommunikationsschnittstellen eingelesen und in dem Datenspeicher 15 langfristig abgespeichert, die Auswertevorrichtung 14 wertet die medizinischen Daten und den Zeitpunkt der Datenübertragung aus, um Informationsnachrichten automatisch versenden zu können. Die Datenübertragung erfolgt beispielsweise über das Internet oder Telefonnetz.

Die Auswertevorrichtung 12 beurteilt, ob die Häufigkeit und Verteilung der Daten einzustufen sind. Als Kriterium werden dabei empirisch ermittelte Grenzen (Sollwerte) genommen, die in dem Datenspeicher 15 abgespeichert sind.

Somit können analog zu regionalen Wetterdiensten, aktuellen Staumeldungen oder Pollenflugmeldungen die Erfassung und Weitergabe von Informationen über die aktuelle Verbreitung von Infektionskrankheiten, wie beispielsweise Grippe, grippale Infekte, Magen-Darmgrippe, Hepatitis, Kinderkrankheiten wie Masern, Mumps, Röteln, Windpocken, Keuchhusten, Scharlach, Diphtherie, Dreitagefieber usw. erfasst und jederzeit abgerufen werden. Durch die Auswertung mittels Vergleich der erfassten Daten kann festgestellt werden, ob es sich um Einzelfälle oder um eine Epidemie handelt.

Die Auswertevorrichtung 14 weist beispielsweise einen Komparator zum Vergleich der Daten mit in dem Datenspeicher 15 gespeicherten Sollwerten und eine Alarmvorrichtung zur Benachrichtigung ausgewählter Empfänger bei Überschreitung der Messwerte von gespeicherten Sollwertgrenzen auf.

Die Erfassung erfolgt direkt durch die betroffenen Patienten oder Angehörige, beispielsweise die Eltern, den behandelnden Ärzten, Apotheken oder sonstigen Verantwortlichen, wie beispielsweise Schulleitung, Arbeitgeber usw. Nutzer der Dienstleistung können via Internet, Telefon, Fax-Polling, Bildschirmtext oder anderen Kommunikationsmechanismen für eine bestimmte oder mehrere Regionen die aktuelle Häufigkeit und Verteilung abfragen. Nutzer der Dienstleistung sind wieder die oben erwähnten Personenkreise und gegebenenfalls weitere Personen oder Unternehmen, die regional gruppierte, tagesaktuelle oder statistische Zahlen benötigen.

Das erfindungsgemäße System weist folgende Vorteile auf:
- Die dezentrale Erfassung erfolgt breit gestreut, regional gruppiert und kostengünstig.
- Die tagesaktuellen Daten können als aktuelle a priori Wahrscheinlichkeiten im laufenden Betrieb in die medizinische Expertensysteme eingegeben werden; damit passen sich die Systeme automatisch an aktuelle Situationen an.

Die medizinischen Daten können
- vom Patienten,
- bei Krankmeldungen von Institutionen, wie beispielsweise Schule, Kindergarten, Arbeitgeber o.ä. sowie
- von ärztlichen Dienststellen, wie beispielsweise Arzt, Apotheke oder Krankenhaus
der Systemzentrale 6 übermittelt werden, wobei beispielsweise das Internet (WWW) oder Telefon (Call-Center 16) eingesetzt werden können.

Die ausgewerteten medizinischen Daten lassen sich vielseitig einsetzen. So können sie beispielsweise zur
- Diagnoseunterstützung für behandelnde Ärzte,
- Präventionsunterstützung für Patienten,
- automatische Verwendung als Verteilungswahrscheinlichkeiten in Expertensystemen,
- Weitergabe kumulierter Daten zu statistischen oder wissenschaftlichen Zwecken (Epidemiologie),
- Unterstützung für Marketing und Logistik der Pharmaindustrie und
- Franchising für regionale Medien, wie beispielsweise Zeitungen, Rundfunk oder Fernsehen
dienen.

Das erfindungsgemäße System dient als Informationsdienst für regional gruppierte, tagesaktuelle medizinische Daten über Häufigkeit und Verteilung von Infektionskrankheiten. Optional lässt sich dieser Informationsdienst zur Parametrisierung medizinischer Expertensysteme mit aktuellen Wahrscheinlichkeiten nutzen.

## Patentansprüche

1. System zur Erfassung und Information von regional gruppierten, tagesaktuellen medizinischen Daten mit Vorrichtungen (4 bis 7) zur Erfassung der medizinischen Daten, mit Vorrichtungen (9 bis 12) zur Übertragung der medizinischen Daten an eine Systemzentrale (8), mit einer Auswertevorrichtung (14) für die medizinischen Daten, mit Vorrichtungen (4 bis 7) zur Abfrage der ausgewerteten medizinischen Daten und mit Vorrichtungen (9 bis 12) zur Übertragung der ausgewerteten medizinischen Daten von der Systemzentrale (8), wobei die Systemzentrale (8) einen Speicher (15) für die erfassten medizinischen Daten und Sollwerte für die medizinischen Daten, und die Auswertevorrichtung (14) einen Komparator zum Vergleich der medizinischen Daten mit den gespeicherten Sollwerten aufweist.

2. System nach Anspruch 1, **dadurch gekennzeichnet, dass** die Auswertevorrichtung (14) ein Expertensystem aufweist.

3. System nach Anspruch 1 oder 2, **dadurch gekennzeichnet, dass** die Vorrichtungen (4 bis 7) zur Erfassung der medizinischen Daten im Hause (1) eines Patienten angeordnet sind.

4. System nach einem der Ansprüche 1 bis 3, **dadurch gekennzeichnet, dass** die Vorrichtungen (4 bis 7) zur Erfassung der medizinischen Daten bei Institutionen (2) angeordnet sind.

5. System nach einem der Ansprüche 1 bis 4, **dadurch gekennzeichnet, dass** die Vorrichtungen (4 bis 7) zur Erfassung der medizinischen Daten bei ärztlichen Dienststellen (3) angeordnet sind.

6. System nach einem der Ansprüche 1 bis 5, **dadurch gekennzeichnet, dass** die Vorrichtungen (4 bis 7) zur Erfassung der medizinischen Daten und/oder der ausgewerteten Daten über das Internet (WWW) mit der Systemzentrale (8) kommunizieren.

7. System nach Anspruch 6, **dadurch gekennzeichnet, dass** die medizinischen Expertensystemen über die Daten aus dem Internet automatisch konfiguriert werden.

8. System nach einem der Ansprüche 1 bis 7, **dadurch gekennzeichnet, dass** die Vorrichtungen (4 bis 7) zur Erfassung der medizinischen Daten und/oder der ausgewerteten Daten mittels eines Telefons (6 oder 7) über ein Call-Center (16) mit der Systemzentrale (8) kommunizieren.
